(19) Europäisches Patentamt
European Patent Office
Office européen des brevets

(11) **EP 3 191 073 B1**

(12) **EUROPEAN PATENT SPECIFICATION**

(45) Date of publication and mention
of the grant of the patent:
**02.03.2022   Bulletin 2022/09**

(21) Application number: **15794683.1**

(22) Date of filing: **21.10.2015**

(51) International Patent Classification (IPC):
*A61K 8/27* $^{(2006.01)}$       *A61Q 11/00* $^{(2006.01)}$
*A61K 8/22* $^{(2006.01)}$

(52) Cooperative Patent Classification (CPC):
**A61K 8/22; A61K 8/27; A61Q 11/00;** A61K 2800/31

(86) International application number:
**PCT/US2015/056768**

(87) International publication number:
**WO 2016/065076 (28.04.2016 Gazette 2016/17)**

(54) **ORAL CARE COMPOSITIONS COMPRISING ZINC COMPOUNDS AND PEROXIDE COMPOUNDS**

MUNDPFLEGEZUSAMMENSETZUNGEN MIT ZINKVERBINDUNGEN UND PEROXIDVERBINDUNGEN

COMPOSITIONS POUR SOINS BUCCAUX COMPRENANT DES COMPOSÉS DE ZINC ET DES COMPOSÉS DE PEROXYDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **23.10.2014   US 201462067950 P**

(43) Date of publication of application:
**19.07.2017   Bulletin 2017/29**

(73) Proprietor: **Colgate-Palmolive Company New York, NY 10022 (US)**

(72) Inventors:
  • **ROBBINS, Kyle**
    **Toms River, New Jersey 08753 (US)**
  • **NESTA, Jason**
    **Somserset, New Jersey 08873 (US)**
  • **CHOPRA, Suman**
    **Monroe, New Jersey 08831 (US)**

(74) Representative: **Wibbelmann, Jobst Wuesthoff & Wuesthoff Patentanwälte PartG mbB Schweigerstrasse 2 81541 München (DE)**

(56) References cited:
**WO-A1-91/11987          WO-A1-96/26707
WO-A1-2014/056212     WO-A1-2014/063662
WO-A1-2014/092735     WO-A1-2014/098828
US-A- 4 226 851           US-A- 5 616 313
US-A1- 2012 148 506**

**Description**

**BACKGROUND**

Peroxide compounds are added to oral care compositions such as dentifrices, mouthrinses, strips and gels to whiten teeth through the bleaching of stains. However, many countries regulate the level of hydrogen peroxide allowable in these products to a maximum of 0.1 weight %. It would be desirable to improve the whitening benefits of these products. WO 2014/092735 A1 discloses oral care compositions comprising less than 3 % water and a peroxide whitening agent.

**BRIEF SUMMARY**

The present invention concerns an oral care composition comprising a peroxide compound, wherein the peroxide compound is hydrogen peroxide present as a complex with polyvinylpyrrolidone, present in a concentration of from 0.01 to 0.5 weight % based on the total weight of the composition, and a zinc compound, wherein the zinc compound is zinc oxide, wherein the weight ratio of the peroxide compound to the zinc compound is from 1:10 to 5:1, and wherein the composition is anhydrous, meaning that the composition contains 2 weight % or less free water.
Further embodiments are set forth in the attached claims.
Optionally, the composition is a toothpaste, a liquid, a gel, or a whitening strip.
In a second aspect, the present invention provides a method of increasing the whitening efficacy of an oral care composition which comprises a peroxide compound wherein the peroxide compound is hydrogen peroxide present as a complex with polyvinylpyrrolidone, present in a concentration of from 0.01 to 0.5 weight % based on the total weight of the composition the method comprising formulating the composition so as to further comprise a zinc compound, wherein the zinc compound is zinc oxide, wherein the oral care composition contains 2 weight % or less free water, wherein the weight ratio of the peroxide compound to the zinc compound is from 1:10 to 5:1
Optionally, the weight ratio of the peroxide compound to the zinc salt is from 1:8 to 4:1, optionally from 1:5 to 3:1, optionally from 1:3 to 2:1, optionally from 1:2 to 1:1, further optionally 1:1.
Optionally, the composition is a toothpaste, a liquid, a gel, or a whitening strip.
In a third aspect, the present invention provides the use, in an oral care composition which comprises a peroxide compound, of a zinc compound to increase the whitening efficacy of the composition, wherein the oral care composition is the inventive composition as outlined above.
Further areas of applicability of the present invention will become apparent from the detailed description provided hereinafter.

**DETAILED DESCRIPTION**

Unless otherwise specified, all percentages and amounts expressed herein and elsewhere in the specification should be understood to refer to percentages by weight. The amounts given are based on the active weight of the material.
All ratios expressed herein should be understood to refer to ratios by weight, unless otherwise indicated.
As discussed above, it would be desirable to provide oral care compositions in which the whitening efficacy is improved, even when relatively low levels of peroxide compound are included.
The present inventors have surprisingly found that the inclusion of a zinc compound in an oral care composition comprising a peroxide compound, at the above ratio of zinc compound to peroxide compound, results in a significant improvement in the whitening efficacy of the composition. This result is particularly surprising, and cannot be explained by any antibacterial activity of the zinc compound or by any change in pH caused by the zinc compound (as discussed in the Examples, below). This discovery allows for use of a lower amount of peroxide compound while maintaining the efficacy of a higher amount of peroxide compound, and the use of a lower amount of peroxide compound with higher efficacy than a peroxide compound alone.
The hydrogen peroxide is present as a complex with a polymer (i.e. the hydrogen peroxide is present as a bound peroxide). The polymer is polyvinylpyrrolidone (PVP). In some embodiments, the complex comprises from 17 to 22 weight % hydrogen peroxide, based on the weight of the complex. In some embodiments, the complex comprises about 18 weight % hydrogen peroxide, based on the weight of the complex.
In some embodiments, the peroxide compound is present in a concentration of from 0.005 to 3 weight %, from 0.01 to 1 weight %, from from 0.01 to 0.5 weight %, from 0.02 to 0.4 weight %, from 0.03 to 0.3 weight %, from 0.04 to 0.2 weight %, from 0.05 to 0.1 weight %, from 0.075 to 0.1 weight %, or from 0.09 to 0.1 weight %, based on the total weight of the composition.
The composition according to the invention is anhydrous. By the composition being "anhydrous", it is meant that the composition contains 2 weight % or less free water. In other embodiments, the maximum amount of water is 1.5 weight %, 1 weight %, or 0.5 weight %. In certain embodiments, the composition contains no free water. When calculating the water content, water molecules that are part of a hydrate of a material are not counted.

In some embodiments, the composition is a toothpaste, a liquid, a gel, a whitening strip, or a composition which is applied to the teeth using a dental tray. In certain embodiments, the composition is a gel. In some embodiments, the gel is adapted to be applied to the teeth by brushing (e.g. in a similar way to toothpaste).

In any of the above embodiments of the oral care composition, method and use, the compositions may further comprise additional ingredients. These additional ingredients may include, but are not limited to, diluents, silicones (such as dimethicone and DC8-7016 silicone fluid), bicarbonate salts, surfactants, sweeteners, flavorants, pigments, anticalculus or tartar control agents, polymers (such as xanthan gum, carboxymethylcellulose, carrageenan gum, polyvinylpyrrolidone) and mixtures thereof.

In some embodiments, the oral care compositions of the present invention comprise at least one bicarbonate salt useful for example to impart a "clean feel" to teeth and gums due to effervescence and release of carbon dioxide. The one or more additional bicarbonate salts are optionally present in a total amount of about 0.1 wt. % to about 50 wt. %, for example about 1 wt. % to 20 wt. %, by total weight of the composition.

The oral care compositions of the invention may also comprise at least one surfactant. Any orally acceptable surfactant, most of which are anionic, nonionic or amphoteric, can be used. One or more surfactants are optionally present in a total amount of about 0.01 wt.% to about 10 wt. %, for example, from about 0.05 wt. % to about 5 wt. %, or from about 0.1 wt. % to about 2 wt. % by total weight of the composition.

The oral care compositions of the present invention may comprise at least one sweetener (such as, for example, sodium saccharin), useful for example to enhance taste of the composition. One or more sweeteners are optionally present in a total amount depending strongly on the particular sweetener(s) selected, but typically 0.005 wt.% to 5 wt.%, by total weight of the composition, optionally 0.005 wt.% to 1 wt.%, further optionally 0.05 wt.% to 0.5 wt.% by total weight of the composition.

The compositions of the present invention may also comprise at least one flavorant, useful for example to enhance taste of the composition. One or more flavorants are optionally present in a total amount of from about 0.01 wt. % to about 5 wt. %, for example, from about 0,03 wt. % to about 2,5 wt.%, optionally about 0.05 wt.% to about 1.5 wt.%, further optionally about 0.1 wt.% to about 1 wt.% by total weight of the composition.

The compositions of the invention may comprise at least one colorant. Colorants herein include pigments, coated dyes, protected dyes, lakes and agents imparting a particular luster or reflectivity such as pearling agents. Any orally acceptable colorant can be used. One or more colorants are optionally present in a total amount of from about 0.00 1 wt.% to about 20 wt.%, for example, from about 0.01 wt.% to about 10 wt. %, or from about 0.1 wt. % to about 5 wt.%, by total weight of the composition.

The compositions of the present invention may comprise a saliva stimulating agent useful, for example, in amelioration of dry mouth. One or more saliva stimulating agents are optionally present in saliva stimulating effective total amount,

The compositions of the present invention may include antisensitivity agents. Such agents may be added in effective amounts, e.g., from about 1 wt. % to about 20 wt. % by weight based on the total weight of the composition, depending on the agent chosen.

## EXAMPLES

Example 1

Two gel compositions were formulated, as shown in Table 1. Both the Comparative and the Example compositions contained 0.55 weight % of a PVP-$H_2O_2$ complex (wherein the concentration of $H_2O_2$ in the complex was 18 weight %, based oii the weight of the complex). The concentration of hydrogen peroxide in each of these compositions was therefore 0.1 weight %, based on the weight of the composition. These compositions differed only in that the Example composition also contained 1 weight % zinc oxide, whereas the Comparative composition contained no zinc oxide (with the Comparative composition containing an additional 1 weight % of one of the non-silicone polymers, in order to make the total weight of the composition up to 100 weight %).

**Table 1**

| Material | Weight % | |
|---|---|---|
| | **Comparative composition** | **Example composition** |
| Silicones | 45.55 | 45.55 |
| Non-silicone polymers | 53.00 | 52.00 |

(continued)

| Material | Weight % | |
|---|---|---|
| | **Comparative composition** | **Example composition** |
| Sweetener and flavor | 0.90 | 0.90 |
| PV P complexed with $H_2O_2$ | 0.55 | 0.55 |
| Zinc oxide | 0.00 | 1.00 |
| **Total** | **100.00** | **100.00** |

Example 2

The whitening efficacy of the Example and Comparative compositions from Table 1, above, was measured in a laboratory test using stained bovine incisors. The incisors were first prepared by brushing with a solution of a peroxide-free toothpaste (which contained no other tooth bleaching agents) for 30 minutes. The $L^*$ value of the incisors was measured using a Spectroshade spectrophotometer and software. Only those incisors having a $L^*$ value of from 60 to 68 were selected for the study. The incisors were then sorted into groups of four, so that the incisors in a particular group had the same $L^*$ value.

Each group of four teeth (mounted so that only the surface of the teeth was exposed) was then subjected to six treatment cycles, with each cycle being two treatment applications (as described below) to mimic twice-daily usage. A first group of four teeth was treated using the Comparative composition from Table 1, above, and a second group of four teeth was treated using the Example composition from Table 1, above. Each treatment application had the following four steps:

(1) Approximately 50 mg of the composition under test was spread evenly across the four teeth.
(2) The teeth were inverted and submerged in a trough containing pH 7,4 aqueous phosphate buffer flowing at a rate of 2 ml/min, for 15 minutes.
(3) The teeth were removed from the trough and any remaining composition was gently removed using a laboratory wipe.
(4) The teeth were inverted and submerged in a trough containing pH 7.4 aqueous phosphate buffer for 15 minutes to rehydrate.

The Spectroshade spectrometer and software were used to measure the $L^*a^*b^*$ values of the teeth which were then used to calculate $\Delta W^*$, which is a common whitening index used for tooth whitening studies. The values shown in Table 2 below are the average of $\Delta W^*$ as measured for each of the four individual teeth in the group. As discussed above, each cycle corresponds to two treatment applications.

**Table 2: whitening efficacy**

| | $\Delta W^*$ | | | | | | |
|---|---|---|---|---|---|---|---|
| | baseline | 1 cycle | 2 cycles | 3 cycles | 4 cycles | 5 cycles | 6 cycles |
| **Comparative composition** | 0 | -0.25 | -0.33 | -0.41 | -0.52 | -0.70 | -0.79 |
| **Example composition** | 0 | -0.64 | -0.96 | -1.16 | -1.29 | -1.48 | -1.62 |

The evaluation of the color of the stained teeth was quantified using measurement of the $L^*a^*b^*$ color space, using a Spectroshade spectrometer and software. $L^*a^*b^*$ refers to stain score in accordance with the Commission International de L'Eclairage Laboratory (CIELAB) color scale, with $L^*$ being lightness-darkness scale, a* being red-green chroma, and b* being yellow-blue chroma. From measurement of the $L^*a^*b^*$ values, the whitening index $\Delta W^*$ was calculated, wherein:

$$\Delta W^* = W^*final - W^*initial$$

and

$$W^* = (a^{*2} + b^{*2} + (L^*-100)^2)^{1/2}.$$

The more negative the value of $\Delta W^*$, the closer the tooth color is to white.

As can be seen from Table 2, above, the whiteness improvement of teeth treated with the Example composition (which contained zinc oxide) was greater than twice that of the teeth treated with the Comparative composition, after 1, 2, 3, 4, 5 and 6 cycles of treatment.

This was a surprising result, particularly given that the increase in whitening efficacy of the Example composition cannot be explained by any antibacterial activity of the zinc oxide (as no bacteria were introduced into either the Example or the Comparative compositions).

Example 3

In accordance with the procedures explained in Example 2, the whitening index used for tooth whitening studies ($\Delta W^*$) were applied to teeth which were independently exposed to both a 1:1 ratio of Hydrogen Peroxide: Zinc (1% Hydrogen Peroxide Gel), as well as a 4.5:1 ratio of Hydrogen Peroxide: Zinc (4.5% Hydrogen Peroxide Gel). Wherein the percent weight of hydrogen peroxide in the gel represents the weight relative to the total composition.

1:1 ratio of HP:Zinc (1% HP gel)

**Table 3**

| Material | Formula Weight % | |
|---|---|---|
| | Comparative composition | Example composition |
| DC8-7016 Silicone Fluid | 30.00 | 30.00 |
| Dimethicone 350 CST | 14.00 | 13.00 |
| Plasticized Hydrocarbon Base | 30.10 | 30.10 |
| Sodium Saccharin | 0.30 | 0.30 |
| Flavor | 0.60 | 0.60 |
| PVP complexed with hydrogen peroxide | 5.50 | 5.50 |
| PVP | 19.5 | 19.5 |
| Zinc oxide | --- | 1.00 |

**Table 4: Whitenning efficacy**

| Group | $\Delta W$ | | | | | | |
|---|---|---|---|---|---|---|---|
| | Baseline | 2 cycles | 4 cycles | 6 cycles | 8 cycles | 10 cycles | 12 cycles |
| Comparative composition | 0 | -2.17 | -3.05 | -3.59 | -3.89 | -4.09 | -4.75 |
| Example Composition | 0 | -2.42 | -3.43 | -3.88 | -4.14 | -4.57 | -5.41 |
| % Imp | N/A | 11.5% | 12.5% | 8.1% | 6.4% | 11.7% | 13.9% |

4.5:1 ratio of HP:Zinc (4.5% HP gel)

**Table 5**

| Material | Formula Weight % | |
|---|---|---|
| | Comparative composition | Example composition |
| DC8-7016 Silicone Fluid | 30.00 | 30.00 |
| Dimethicone 350 CST | 12.00 | 13.00 |
| Plasticized Hydrocarbon Base | 30.10 | 30.10 |
| Sodium Saccharin | 0.30 | 0.30 |
| Flavor | 0.60 | 0.60 |
| PVP complexed with hydrogen peroxide | 25.00 | 25.00 |
| Zinc oxide | - | 1.00 |

**Table 6: Whitening efficacy**

| Group | A W | | | | | | |
|---|---|---|---|---|---|---|---|
| | Baseline | 2 cycles | 4 cycles | 6 cycles | 8 cycles | 10 cycles | 12 cycles |
| Comparative Composition | 0 | -4.28 | -6.19 | -7.02 | -7.89 | -8.50 | -9.25 |
| Example Composition | 0 | -5.04 | -6.85 | -8.00 | -9.13 | -10.07 | -11.01 |
| % Imp | N/A | 10.7% | 10.7% | 14.0% | 15.7% | 18.5% | 19.0% |

Similar to the results demonstrated in Example 2, these figures represent surprising results. And the increase in whitening efficacy of the Example composition cannot be explained by the antibacterial activity of the zinc oxide (as no bacteria were introduced into either the Example or the Comparative compositions).

Example 3

An experiment was conducted to determine whether or not the zinc oxide present in the Example composition affected the pH of saliva. For each of the Example and Comparative compositions in Table 1, above, a slurry was prepared containing 10 weight % of the composition dispersed in artificial saliva solution. Three aliquots of each solution were taken after 10, 20 and 30 minutes of agitation. The aliquots were centrifuged and the pH of the supernatant was measured. The results are shown in Table 7, below (average of the pH measured for the three aliquots).

**Table 7: Impact on pH**

| | pH of supernatant | | | |
|---|---|---|---|---|
| | baseline | 10 minutes | 20 minutes | 30 minutes |
| **Comparative composition** | 6.71 | 6.91 | 6.98 | 7.11 |
| **Example composition** | 6.71 | 6.84 | 6.94 | 7.56 |

As can be seen from Table 7, above, no significant difference in pH was observed between the Example composition and the Comparative composition within the treatment time as used for each cycle of the whitening experiment in Example 2, above. The lack of any significant pH difference between the Example and Comparative compositions at all time periods during the whitening cycle makes the increased whitening efficacy of the Example composition particularly surprising, as this effect cannot be attributed to any difference in pH between the two compositions. Without being bound by any theory, it is hypothesized that the increased whitening efficacy observed for the zinc oxide-containing composition might be due to the zinc oxide stabilizing the reactive oxygen species of hydrogen peroxide for oxidation.

**Claims**

1. An oral care composition comprising a peroxide compound, wherein the peroxide compound is hydrogen peroxide present as a complex with polyvinylpyrrolidone, present in a concentration of from 0.01 to 0.5 weight % based on the total weight of the composition, and a zinc compound, wherein the zinc compound is zinc oxide, wherein the weight ratio of the peroxide compound to the zinc compound is from 1:10 to 5:1, and wherein the composition is anhydrous, meaning that the composition contains 2 weight % or less free water.

2. The oral care composition of claim 1, wherein the weight ratio of the peroxide compound to the zinc compound is from 1:8 to 4:1.

3. The oral care composition of claim 2, wherein the weight ratio of the peroxide compound to the zinc compound is from 1:5 to 3:1.

4. The oral care composition of claim 3, wherein the weight ratio of the peroxide compound to the zinc compound is from 1:3 to 2:1.

5. The oral care composition of claim 4, wherein the weight ratio of the peroxide compound to the zinc compound is 1:1.

6. The oral care composition of any preceding claim, wherein the zinc compound is present in a concentration of from 0.1 to 5 weight %, optionally from 0.75 to 1.5 weight %, based on the weight of the composition.

7. The oral care composition of any preceding claim, wherein the peroxide compound is present in a concentration from 0.05 to 0.1 weight %, based on the total weight of the composition.

8. The oral care composition of any preceding claim, wherein the composition is a dentifrice, wherein the dentifrice is a toothpaste, gel, or a whitening strip.

9. A method of increasing the whitening efficacy of an oral care composition which comprises a peroxide compound, wherein the peroxide compound is hydrogen peroxide present as a complex with polyvinylpyrrolidone, present in a concentration of from 0.01 to 0.5 weight % based on the total weight of the composition the method comprising formulating the composition so as to further comprise a zinc compound, wherein the zinc compound is zinc oxide, wherein the oral care composition contains 2 weight % or less free water and wherein the weight ratio of the peroxide compound to the zinc compound in the composition is from 1:10 to 5:1.

10. Use, in an oral care composition which comprises a peroxide compound, of a zinc compound to increase the whitening efficacy of the composition, wherein the oral care composition is according to any of the preceding claims 1-8.

**Patentansprüche**

1. Mundpflegezusammensetzung, umfassend eine Peroxidverbindung, wobei es sich bei der Peroxidverbindung um Wasserstoffperoxid handelt, das als Komplex mit Polyvinylpyrrolidon vorliegt und in einer Konzentration von 0,01 bis 0,5 Gew.-%, bezogen auf das Gesamtgewicht der Zusammensetzung, vorliegt, und eine Zinkverbindung, wobei es sich bei der Zinkverbindung um Zinkoxid handelt, wobei das Gewichtsverhältnis der Peroxidverbindung zur Zinkverbindung 1:10 bis 5:1 beträgt und wobei die Zusammensetzung wasserfrei ist, was bedeutet, dass die Zusammensetzung 2 Gew.-% oder weniger freies Wasser enthält.

2. Mundpflegezusammensetzung nach Anspruch 1, wobei das Gewichtsverhältnis der Peroxidverbindung zur Zinkverbindung 1:8 bis 4:1 beträgt.

3. Mundpflegezusammensetzung nach Anspruch 2, wobei das Gewichtsverhältnis der Peroxidverbindung zur Zinkverbindung 1:5 bis 3:1 beträgt.

4. Mundpflegezusammensetzung nach Anspruch 3, wobei das Gewichtsverhältnis der Peroxidverbindung zur Zinkverbindung 1:3 bis 2:1 beträgt.

**5.** Mundpflegezusammensetzung nach Anspruch 4, wobei das Gewichtsverhältnis der Peroxidverbindung zur Zinkverbindung 1:1 beträgt.

**6.** Mundpflegezusammensetzung nach einem der vorhergehenden Ansprüche, wobei die Zinkverbindung in einer Konzentration von 0,1 bis 5 Gew.-%, gegebenenfalls von 0,75 bis 1,5 Gew.-%, bezogen auf das Gewicht der Zusammensetzung, vorhanden ist.

**7.** Mundpflegezusammensetzung nach einem der vorhergehenden Ansprüche, wobei die Peroxidverbindung in einer Konzentration von 0,05 bis 0,1 Gew.-%, bezogen auf das Gesamtgewicht der Zusammensetzung, vorhanden ist.

**8.** Mundpflegezusammensetzung nach einem der vorhergehenden Ansprüche, wobei die Zusammensetzung ein Zahnpflegemittel ist, wobei das Zahnpflegemittel eine Zahnpasta, ein Gel oder ein Aufhellungsstreifen ist.

**9.** Verfahren zur Erhöhung der Aufhellungswirkung einer Mundpflegezusammensetzung, die eine Peroxidverbindung umfasst, wobei die Peroxidverbindung Wasserstoffperoxid ist, das als Komplex mit Polyvinylpyrrolidon vorliegt, das in einer Konzentration von 0,01 bis 0.5 Gew.-%, bezogen auf das Gesamtgewicht der Zusammensetzung, vorliegt, wobei das Verfahren das Formulieren der Zusammensetzung umfasst, so dass sie ferner eine Zinkverbindung umfasst, wobei die Zinkverbindung Zinkoxid ist, wobei die Mundpflegezusammensetzung 2 Gew.-% oder weniger freies Wasser enthält und wobei das Gewichtsverhältnis der Peroxidverbindung zu der Zinkverbindung in der Zusammensetzung 1:10 bis 5:1 beträgt.

**10.** Verwendung einer Zinkverbindung in einer Mundpflegezusammensetzung, die eine Peroxidverbindung enthält, um die Aufhellungswirkung der Zusammensetzung zu erhöhen, wobei die Mundpflegezusammensetzung einem der vorhergehenden Ansprüche 1-8 entspricht.

## Revendications

**1.** Composition de soin bucco-dentaire comprenant un composé peroxyde, dans laquelle le composé peroxyde est du peroxyde d'hydrogène présent sous forme d'un complexe avec de la polyvinylpyrrolidone, présent à une concentration de 0,01 à 0,5 % en poids par rapport au poids total de la composition, et un composé de zinc, dans lequel le composé de zinc est l'oxyde de zinc, dans laquelle le rapport pondéral du composé peroxyde au composé de zinc est de 1:10 à 5:1, et dans laquelle la composition est anhydre, ce qui signifie que la composition contient 2 % en poids ou moins d'eau libre.

**2.** Composition de soin bucco-dentaire selon la revendication 1, dans laquelle le rapport pondéral du composé peroxyde au composé de zinc est de 1:8 à 4:1.

**3.** Composition de soin bucco-dentaire selon la revendication 2, dans laquelle le rapport pondéral du composé peroxyde au composé de zinc est de 1:5 à 3:1.

**4.** Composition de soin bucco-dentaire selon la revendication 3, dans laquelle le rapport pondéral du composé peroxyde au composé de zinc est de 1:3 à 2:1.

**5.** Composition de soin bucco-dentaire selon la revendication 4, dans laquelle le rapport pondéral du composé peroxyde au composé de zinc est de 1:1.

**6.** Composition de soin bucco-dentaire selon l'une quelconque des revendications précédentes, dans laquelle le composé de zinc est présent à une concentration de 0,1 à 5 % en poids, éventuellement de 0,75 à 1,5 % en poids, par rapport au poids de la composition.

**7.** Composition de soin bucco-dentaire selon l'une quelconque des revendications précédentes, dans laquelle le composé peroxyde est présent à une concentration de 0,05 à 0,1 % en poids, par rapport au poids total de la composition.

**8.** Composition de soin bucco-dentaire selon l'une quelconque des revendications précédentes, dans laquelle la composition est un dentifrice, dans laquelle le dentifrice est une pâte dentifrice, un gel ou une bandelette de blanchiment.

**9.** Procédé pour augmenter l'efficacité de blanchiment d'une composition de soin bucco-dentaire qui comprend un composé peroxyde, dans lequel le composé peroxyde est du peroxyde d'hydrogène présent sous forme d'un complexe avec de la polyvinylpyrrolidone, présent à une concentration de 0,01 à 0,5 % en poids par rapport au poids total de la composition, le procédé comprenant la formulation de la composition de manière à comprendre en outre un composé de zinc, dans lequel le composé de zinc est l'oxyde de zinc, dans lequel la composition de soin bucco-dentaire contient 2 % en poids ou moins d'eau libre et dans lequel le rapport pondéral du composé peroxyde au composé de zinc dans la composition est de 1:10 à 5:1.

10. Utilisation, dans une composition de soin bucco-dentaire qui comprend un composé peroxyde, d'un composé de zinc pour augmenter l'efficacité de blanchiment de la composition, dans laquelle la composition de soin bucco-dentaire est selon l'une quelconque des revendications 1 à 8 précédentes.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014092735 A1